# EUROPEAN PATENT SPECIFICATION

(11) **EP 2 943 082 B1**
(45) Date of publication and mention of the grant of the patent: **12.04.2017**
(21) Application number: 13703647.1
(22) Date of filing: 09.01.2013
(51) Int. Cl.: A23L 33/175, A23L 33/00, A61K 38/05, A61K 47/26, A61K 38/06, A61K 31/198

(54) **IMPROVEMENT OF COGNITIVE FUNCTION IN PRETERM INFANTS THAT ARE SMALL FOR GESTATIONAL AGE**
VERBESSERUNG DER KOGNITIVEN FUNKTION BEI FRÜHGEBORENEN MIT KLEINER GRÖSSE FÜR DAS GESTATIONSALTER
AMÉLIORATION DE LA FONCTION COGNITIVE CHEZ DES NOURRISSONS NÉS AVANT TERME ET SE RÉVÉLANT PETITS POUR LEUR ÂGE GESTATIONNEL

(43) Date of publication of application: 18.11.2015
(73) Proprietor: N.V. Nutricia, 2712 HM Zoetermeer (NL)
(72) Inventor: VAN ELBURG, Roelof Matthijs, NL-3584 CT Utrecht (NL)
(74) Representative: Nederlandsch Octrooibureau
(86) International application number: PCT/NL2013/050009
(87) International publication number: WO 2014/109632

(56) References cited:
- WO-A1-2011/047107
- DE KIEVIET JORRIT F ET AL: "Effects of neonatal enteral glutamine supplementation on cognitive, motor and behavioural outcomes in very preterm and/or very low birth weight children at school age", December 2012 (2012-12), BRITISH JOURNAL OF NUTRITION, VOL. 108, NR. 12, PAGE(S) 2215-2220, XP009168040, ISSN: 0007-1145(print) the whole document
- PHILLIPS JOHN P ET AL: "Anterior Cingulate and Frontal Lobe White Matter Spectroscopy in Early Childhood of Former Very LBW Premature Infants", March 2011 (2011-03), PEDIATRIC RESEARCH, VOL. 69, NR. 3, PAGE(S) 224-229, XP002693883, ISSN: 0031-3998 the whole document

## Description

### FIELD OF THE INVENTION

The present invention is in the field of infant nutrition and relates to providing nutrition to infants that are born preterm and that are small for gestational age (SGA) and in particular the invention relates to the long term beneficial cognitive effect in such preterm-SGA infants.

### BACKGROUND OF THE INVENTION

With advances in neonatal intensive care, the survival of very preterm (born <32 weeks of gestation) and very low birth weight (VLBW; weighing <1500 grams) children has improved considerably. However, a variety of risk factors associated with preterm and low birth weight birth, including neonatal infections and inflammatory responses, adversely affect normal brain maturation processes in these children. As a consequence, widespread differences in brain development compared to term peers are found, as indicated by an overall reduction in grey and white matter volumes as measured by magnetic resonance imaging (MRI) as well as reduced white matter integrity as measured by diffusion tensor imaging (DTI). Unfortunately, these unfavourable differences in brain development appear to pertain into childhood and adolescence, and increase the risks for poor motor, cognitive, and behavioural development in very preterm/VLBW children.

Many infant formulae for term infants are available on the market. However, these are not optimal for administration to preterm infants or infants with a low birth weight, since the complete nutritional needs of a rapidly growing preterm infant are not met. Likewise, the composition of human milk does not fully meet the nutritional requirements. The major goal of enteral nutrient supply to these infants is to achieve growth similar to fetal growth coupled with satisfactory functional development. The preferred food for infants with a low birth weight is therefore fortified human milk, or, alternatively, formula specially designed for these infants. Human milk fortifiers (HMF) and preterm infant formulae are commercially available. Typically, HMF comprises besides protein, also carbohydrates or fat, minerals and vitamins. Typically, preterm infant formulae have an increased protein concentration. Enteral feeding of very low birth weight (VLBW) infants and small for gestational age infants is a challenge, since metabolic demands are high and administration of enteral nutrition is limited by immaturity of the gastrointestinal tract.

The amino acid glutamine has been used in nutritional compositions for preterm infants. Van den Berg et al, 2005, Am J Clin Nutr 81:1397-1404 disclose that supplementation with glutamine lowers infectious morbidity in VLBL infants. Van den Berg et al, 2007, Arch. Pediatrics & Adolescent Medicine, 161:1095-1101 disclose that supplementation with glutamine decreases risk for atopic dermatitis in the first year of life and at the age of 6. Van Zwol et al, 2008, Acta Paediatrica, 97:562-567 report that there is no effect of glutamine supplementation in VLBW infants on neurodevelopment outcome at age of 2 years. Kievet et al. disclose the effects of neonatal enteral glutamine supplementation on cognitive outcomes in children (British Journal of Nutrition, 2012, 108, 2215-2220). Smaller total brain volumes, white and grey matter volumes, as well as smaller volumes of the cerebellum, hippocampus, and corpus callosum have been found associated with lower outcomes in terms of IQ, see Allin et al., Brain 2001, 1: 60-6; Taylor et al., Dev Neuropsychol 2011, 1: 96-117; Parker et al., Brain 2008, 5: 1344-51; Allin et al., Arch Pediatr Adolesc Med 2007, 12: 1183-9; Martinussen et al., J Pediatr 2009, 6: 848-53; Narberhaus et al., Neuropsychologia 2008, 1: 111-6; Reiss et al., J Pediatr 2004, 2:242-9; Yung et al., Pediatr Res 2007, 6: 732-6; Northam et al., Ann Neurol 2011, 4: 702-11.

WO 2011/047107 discloses the use of an arginine-glutamine dipeptide to support retinal, intestinal and/or nervous system development in a neonate.

### SUMMARY OF THE INVENTION

The inventors found that glutamine enriched feeding in the first month after birth in very preterm and/or VLBW infants has a long term effect on measures of brain development. In total 52 very preterm and/or VLBW children, who originally took part in a randomized controlled trial on enteral glutamine supplementation between day 3 and 30 of life, participated in this follow up study at school age, with a mean age of 8.6 years (SD = 0.3 years). Measures of brain development included volumetric outcomes of various brain structures as well as fractional anisotropy (FA) values of major white matter tracts, using Magnetic Resonance Imaging (MRI) including Diffusion Tensor Imaging (DTI) techniques, respectively.

An increased cortical brain volume and subcortical brain volume was observed in children who were born preterm and were supplemented in the first month after birth with L-glutamine when compared to the control group of preterm infants not having received L-glutamine. In particular a significant increased white matter volume, brain stem volume, and hippocampus volume was observed

Surprisingly it was found that glutamine enriched feeding in the first month after birth in preterm infants that were born small for gestational age has a long term effect on improving cognitive function. In particular an improved IQ later in life was measured in preterm infants that were born small for gestational age that were fed glutamine enriched feeding compared to a control group of preterm infants that were born small for gestational age that not received glutamine enriched feeding.

### DETAILED DESCRIPTION OF THE INVENTION

The present subject-matter thus concerns a method for improving cognitive functioning in a preterm infant born small for gestational age, comprising administering a glutamine enriched nutritional composition to said preterm infant born small for gestational age.

The present subject-matter also concerns a method for improving IQ in a preterm infant born small for gestational age, comprising administering a glutamine enriched nutritional composition to said preterm infant born small for gestational age.

In one embodiment, the present subject-matter is considered to be a non-medical method for improving cognitive functioning and/or improving IQ.

Preferably the glutamine enriched nutritional composition is administered during the first six, preferably the first four, more preferably the first two and most preferably at least the first month after birth of the preterm infant born small for gestational age.

Preferably the improvement in cognitive functioning and/or improvement in IQ is established in the infant later in life, preferably at an age above 4 years, more preferably at an age between 4 to 12 years.

The present subject-matter can also be worded as a nutritional composition comprising glutamine for use in improving cognitive functioning later in life, preferably at an age above 4 years, more preferably at an age from 4 to 12 years in a preterm infant born small for gestational age.

The present subject-matter can also be worded as a nutritional composition comprising glutamine for use in improving IQ later in life, preferably at an age above 4 years, more preferably at an age from 4 to 12 years in a preterm infant born small for gestational age.

Preferably the nutritional composition comprising glutamine is for use in providing nutrition to a preterm infant born small for gestational age. The nutritional composition comprising glutamine is for use in providing nutrition to a very preterm infant born small for gestational age.

More in particular the present subject-matter concerns a nutritional composition comprising glutamine for use in providing nutrition to a preterm infant born small for gestational age for use in improving cognitive functioning IQ later in life, preferably at an age above 4 years, more preferably at an age from 4 to 12 years.

Also, more in particular the present subject-matter concerns a nutritional composition comprising glutamine for use in providing nutrition to a preterm infant born small for gestational age for use in improving IQ later in life, preferably at an age above 4 years, more preferably at an age from 4 to 12 years.

The present subject-matter can also be worded as the use of glutamine or of a composition comprising glutamine, for the manufacture of a glutamine enriched nutritional composition for use in providing nutrition to a preterm infant born small for gestational age, for use in improving cognitive functioning IQ later in life, preferably at an age above 4 years, more preferably at an age from 4 to 12 years.

Further the present subject-matter can also be worded as the use of glutamine or of a composition comprising glutamine, for the manufacture of a glutamine enriched nutritional composition for use in providing nutrition to a preterm infant born small for gestational age, for use in improving IQ later in life, preferably at an age above 4 years, more preferably at an age from 4 to 12 years.

According to the present subject-matter improvement in cognitive functioning and/or improvement in IQ in a preterm infant born small for gestational age is with respect to cognitive functioning and/or IQ in preterm infants born small for gestational age not having been administered the glutamine enriched nutritional composition.

### Preterm infants that are born small for gestational age

The present invention relates to a method for feeding preterm infants that are born small for gestational age. Preterm infants are infants that are born before the end of the 37th week of pregnancy. Very preterm infants are born before the end of the 32th week of pregnancy. Small for gestational age (SGA) infants are those whose birth weight lies below the 10th percentile for that gestational age. This is a different (sub)group from low-birth weight (LBW) or very low birth weight (VLBW) children which latter groups also encompass children that are appropriate for gestational age (AGA). According to the invention the preterm infant born small for gestational age is a very preterm infant born small for gestational age.

### Glutamine

The nutritional composition of the present invention comprises glutamine. Throughout this description this is also referred to as nutritional composition comprising glutamine or glutamine enriched nutritional composition. Glutamine in the present invention refers to L-glutamine. Glutamine is one of the most abundant amino acids in plasma and human milk and is considered conditionally essential in preterm infants. Glutamine is utilized as a source of energy and for nucleotide synthesis in all rapidly dividing cells, such as the intestinal lining and certain immune cells. In the brain, glutamine is a substrate for neurotransmitters and an important source of energy for the nervous system. Preterm infants that are born small for gestational age may be especially susceptible to glutamine depletion as nutritional supply of glutamine is limited in the first weeks after birth. Furthermore the endogenous capacity to synthesize glutamine from glutamate is not fully developed in these infants.

Glutamine is preferably present in an easily absorbable form. Because of the immaturity of the intestinal tract of preterm infants that are born small for gestational age, glutamine present in intact protein is less easily absorbed. Therefore the glutamine is preferably present in the form of free amino acids and/or di- and/or tripeptides, most preferably glutamine dipeptide and/or free glutamine. Free glutamine and glutamine dipeptide are commercially available, for example at Ajinomoto, USA. In one embodiment glutamine dipeptide is in the form of a dipeptide of 2 glutamine residues. In one embodiment, glutamine dipeptide is in the form of a dipeptide of one glutamine and one amino acid other than glutamine. For example glutamine dipeptide can also be in the form of alanine-glutamine dipeptide. The term free glutamine in the context of this invnetion means in the form of a single amino acid. This includes glutamine as a single amino acid in the form of a salt.

The nutritional composition of the present invention comprises glutamine levels higher then normally present in human milk protein or standard preterm formula based on cow's milk derived protein. Preferably the nutritional composition of the present invention comprises at least 12 wt. %, more preferably at least 15 wt. %, more preferably at least 20 wt. %, even more preferably at least 30 wt.% glutamine based on total protein. Preferably the nutritional composition of the present invention comprises at least 1.5 wt.%, more preferably at least 2 wt.%, even more preferably at least 4 wt.% glutamine based on dry weight of the nutritional composition. Preferably the nutritional composition of the present invention comprises at least 0.3 g, more preferably at least 0.5 g, even more preferably at least 1 g glutamine based on 100 kcal. Preferably the nutritional composition of the present invention comprises at least 12 wt. %, more preferably at least 15 wt. %, more preferably at least 20 wt. %, even more preferably at least 30 wt.% glutamine in the form of free amino acid and/or dipeptide based on total protein. Preferably the nutritional composition of the present invention comprises at least 1.5 wt.%, more preferably at least 2 wt.%, even more preferably at least 4 wt.% glutamine in the form of free amino acid and/or dipeptide based on dry weight of the nutritional composition. Preferably the nutritional composition of the present invention comprises at least 0.3 g, more preferably at least 0.5 g, even more preferably at least 1 g glutamine in the form of free amino acid and/or dipeptide based on 100 kcal.

The daily dose of glutamine, preferably free glutamine, based on kg body weight, is preferably of 0.01 to 0.5 g, more preferably 0.03 to 0.4 g, even more preferably 0.07 to 0.35 g.

### Nutritional composition in the present method or use

In one embodiment the glutamine enriched composition for use according to the present invention is a nutritional composition. In one embodiment, the present invention relates to nutritional compositions for use in feeding very preterm infants that are born small for gestational age.

In one embodiment the glutamine enriched nutritional composition for use according to the present invention is a preterm formula. The preterm formula comprises all macro- and micronutrients needed for preterm infants so as to achieve a growth similar to fetal growth coupled with satisfactory functional development.

In one embodiment according to the invention the nutritional composition comprises between 1.5 wt.% and 20 wt.% glutamine based on dry weight of the nutritional composition, and comprises protein in such an amount that glutamine is present between 12 wt.% and 80 wt% based on total protein. Preferably the nutritional composition is a preterm formula. Preferably the glutamine is in the form of free glutamine, glutamine dipeptide and/or glutamine tripeptide.

In a preferred embodiment the preterm formula comprises from 5 to 25 wt.% protein, preferably 9 to 20 wt.%, more preferably 13 to 18 wt.% protein based on the dry weight of the preterm formula. In a preferred embodiment the preterm formula comprises from 1.8 to 3.0 g protein, preferably, preferably 2.0 to 3.0 g, preferably 2.5 g to 2.6 g protein, per 100 ml.

In a preferred embodiment according to the invention the preterm formula comprises from 6 to 30 % protein, more preferably 10 to 20, even more preferably 12 to 15 % protein, based on total calories. The wording "% protein based on total calories" refers to the energy of 1 g protein being 4.0 kcal. For determining the amount of protein, the sum of proteins, peptides and free amino acids, including the glutamine, should be taken into account. Preferably, the protein content is determined according to the Kjeldahl method using a conversion factor of 6.25 and preferably using a conversion factor of 6.38 in case of casein dominant formula.

Whey protein is highly suitable as a protein source for preterm infants that are born small for gestational age. However, since sweet whey protein generally has a high threonine content, which can result in hyperthreoninemia, preferably the preterm formula in the present method or use, comprises acid whey protein or sweet whey protein from which at least part of the glycomacropeptide (GMP) is removed. The preterm formula in the present method or use, preferably contains from 4.7 to 7.5 g threonine per 100 g amino acids, preferably from 3.9 to 4.6 g threonine per 100 g amino acids. Preferably, the preterm formula in the present method or use, contains non-hydrolysed protein, in order to decrease the osmolarity of the reconstituted preterm formula, which is beneficial in preterm infants that are born small for gestational age. Advantageously, the protein of the preterm formula in the present method or use is hydrolysed in order to increase digestibility and gastrointestinal tolerance. This is particularly preferred in preterm infants that are born small for gestaional age since they have an immature intestinal tract which impaired digestion and absorption capacity. Advantageously and preferably, the protein is present in the preterm formula in partially hydrolyzed form, thereby enabling a better solubility and digestion than intact protein, without increasing the osmolarity too much, as is the case with extensively hydrolysed protein or free amino acids. Partially hydrolyzed protein in this invention refers to protein with a degree of hydrolysis from 3 to 20 %. In a preferred embodiment of the invention the preterm formula comprises a protein with a degree of hydrolysis from 3 to 20 %, preferably 5 to 18 %, most preferably 5 to 15 %.

In a preferred embodiment of the invention the preterm formula comprises casein and/or whey protein. In a preferred embodiment of the invention the preterm formula comprises casein. In a preferred embodiment of the invention the preterm formula comprises whey protein. Preferably, the preterm formula comprises whey protein and casein derived from non-human milk, preferably cow's milk. Preferably, the weight ratio (based on dry weight) of casein to whey protein is from 80/20 to 20/80, more preferably from 40/60 to 50/50.

Preferably the preterm formula in the method or use according to the present invention comprises at least 12 wt.%, more preferably at least 15 wt.%, more preferably at least 20 wt.%, even more preferably at least 30 wt.% glutamine based on total protein. Preferably the preterm formula comprises no more than 80 wt.%, more preferably no more than 50 wt.% glutamine based on total protein. Preferably the preterm formula in the present method or use comprises at least 1.5 wt.%, more preferably at least 2 wt.%, even more preferably at least 4 wt.% glutamine based on dry weight of the preterm formula. Preferably the preterm formula comprises no more than 20 wt.%, more preferably no more than 10 wt.% glutamine based on dry weight of the preterm formula. Preferably the preterm formula in the present method or use comprises at least 0.3 g, more preferably at least 0.5 g, even more preferably at least 1 g glutamine based on 100 kcal of the preterm formula. Preferably the preterm formula comprises no more than 5 g, even more preferably no more than 2 g glutamine based on 100 kcal of the preterm formula.

Preferably the preterm formula in the method or use according to the present invention comprises at least 12 wt.%, more preferably at least 15 wt.%, more preferably at least 20 wt.%, even more preferably at least 30 wt.% glutamine in the form of free amino acid and/or dipeptide based on total protein. Preferably the preterm formula comprises no more than 80 wt.%, more preferably no more than 50 wt.% glutamine in the form of free amino acid and/or dipeptide based on total protein. Preferably the preterm formula in the present method or use comprises at least 1.5 wt.%, more preferably at least 2 wt.%, even more preferably at least 4 wt.% glutamine in the form of free amino acid and/or dipeptide based on dry weight of the preterm formula. Preferably the preterm formula comprises no more than 20 wt.%, more preferably no more than 10 wt% glutamine in the form of free amino acid and/or dipeptide based on dry weight of the preterm formula. Preferably the preterm formula in the present method or use comprises at least 0.3 g, more preferably at least 0.5 g, even more preferably at least 1 g glutamine in the form of free amino acid and/or dipeptide based on 100 kcal of the preterm formula. Preferably the preterm formula comprises no more than 5 g, even more preferably no more than 2 g glutamine in the form of free amino acid and/or dipeptide based on 100 kcal of the preterm formula.

The preterm formula in the present method or use in ready to drink form has in a preferred embodiment about 70 to 90 kcal, preferably 75 to 85 kcal per 100 ml.

Preferably the preterm formula has an osmolarity below 450 mOsmol/l, more preferably below 400, even more preferably below 350. Particularly in preterm infants that are born small for gestaional age, a too high osmloartiy is a disadvantage.

In the present method or use, preferably the preterm formula comprises about 3.5 to 5.5 g, preferably 3.9 to 5.0 g, fat per 100 ml. Preferably the preterm formula comprises about 20 to 50 wt.%, preferably 26 to 35 wt.% fat based on dry weight of the preterm formula. Preferably the preterm formula comprises about 3.5 to 7.0 g, more preferably 5.0 to 6.5 g, more preferably 5.3 to 5.8 g fat, based on 100 kcal of the preterm formula. Fat is preferably vegetable fat. Preferably the fat component comprises long chain poly-unsaturated fatty acids such as docosahexaenoic acid and arachidonic acid. These fatty acids are beneficial for brain and visual development in preterm infants that are born small for gestaional age. Preferably the fat component comprises medium chain fatty acids, preferably at least 10 wt% and at most 40 wt% based on total fatty acids. Medium chain fatty acids are more easily absorbed by preterm and/or LBW infants.

In the present method or use, preferably the preterm formula comprises 6 to 10 g, preferably 7 to 8 g, digestible carbohydrates per 100 ml. Preferably the preterm formula comprises 40 to 80 wt.% preferably 40 to 50 wt.% digestible carbohydrates based on dry weight of the preterm formula. Preferably the preterm formula comprises about 5 to 20 g, more preferably 6 to 15 g, more preferably 7 to 10 g digestible carbohydrates, based on 100 kcal of the preterm formula. Suitable digestible carbohydrates are lactose and maltodextin.

In the present method or use, preferably the preterm formula comprises at least one non-digestible oligosaccharide. Advantageously and most preferred, the non-digestible oligosaccharide is water-soluble (according to the method disclosed in L. Prosky et al, J. Assoc. Anal. Chem 71: 1017-1023, 1988). Non-digestible oligosaccharides, also called prebiotics, are not digested in the intestine by the action of digestive enzymes present in the human upper digestive tract (small intestine and stomach) but instead are fermented by the human intestinal microbiota. For example, glucose, fructose, galactose, sucrose, lactose, maltose and the maltodextrins are considered digestible. Non-digestible oligosaccharides advantageously further improve the effect of the present glutamine on structural brain volume or growth. Non-digestible oligosaccharides stimulate a healthy gut environment, thereby aiding the uptake of glutamine.

Preferably the non-digestible oligosaccharide has a degree of polymerisation (DP) of 2 to 200. The average DP of the non-digestible oligosaccharide is preferably below 200, more preferably below 100, even more preferably below 60, most preferably below 40. The non-digestible oligosaccharide is preferably selected from the group consisting of fructo-oligosaccharide, such as inulin, non-digestible dextrin, galacto-oligosaccharide, such as transgalacto-oligosaccharide, xylo-oligosaccharide, arabino-oligosaccharide, arabinogalacto-oligosaccharide, gluco-oligosaccharide, such as gentio-oligosaccharide and cyclodextrin, glucomanno-oligosaccharide, galactomanno-oligosaccharide, mannan-oligosaccharide, chito-oligosaccharide, sialyloligosaccharide, such as 3-SL, 6-SL, LSTa,b,c, DSLNT, S-LNH, DS-LNH, and fuco-oligosaccharide, such as (un)sulphated fucoidan OS, 2-FL, 3-FL, LNFP I, II, III, V, LNnFPI, LNDH, and mixtures thereof, more preferably fructo-oligosaccharide, such as inulin, galacto-oligosaccharide, such as transgalacto-oligosaccharide, which is β-linked, and fuco-oligosaccharide and mixtures thereof, even more preferably transgalacto-oligosaccharide. When the non-digestible oligosaccharide is a mixture, the averages of the respective parameters are used for defining the present invention.

In the present method or use, preferably the composition comprises two different non-digestible oligosaccharides, with a different type of glycosidic linkage, a different degree of polymerisation and/or a different monosaccharide composition. Here below the two different non-digestible oligosaccharides are also referred to as non-digestible oligosaccharide A and B.

Preferably, at least 60%, more preferably at least 75% even more preferably 90%, most preferably 98% of the total monosaccharide units of the non-digestible oligosaccharide, are monosaccharides selected from the group consisting of galactose (gal), fructose (fru) and glucose (glu) monosaccharides.

Preferably the non-digestible oligosaccharide is an oligosaccharide selected from the group consisting of β-galacto-oligosaccharide, α-galacto-oligosaccharide, and galactan. According to a more preferred embodiment non-digestible oligosaccharide A is β-galacto-oligosaccharide. β-galacto-oligosaccharide is also sometimes referred to as transgalacto-oligosaccharide. Preferably non-digestible oligosaccharide A comprises galacto-oligosaccharides with β(1,4), β(1,3) and/or β(1,6) glycosidic bonds and a terminal glucose. Transgalacto-oligosaccharide is for example available under the trade name Vivinal®GOS (Borculo Domo Ingredients, Zwolle, Netherlands), Bi2muno (Clasado), Cup-oligo (Nissin Sugar) and Oligomate55 (Yakult).

Preferably non-digestible oligosaccharide is a fructo-oligosaccharide. A fructo-oligosaccharide may in other context have names like fructopolysaccharides, oligofructose, polyfructose, polyfructan, inulin, levan and fructan and may refer to oligosaccharides comprising β-linked fructose units, which are preferably linked by β(2,1) and/or β(2,6) glycosidic linkages, and a preferable DP between 2 and 200. Preferably, the fructo-oligosaccharide contains a terminal β(2,1) glycosidic linked glucose. Preferably, the fructo-oligosaccharide contains at least 7 β-linked fructose units. In a further preferred embodiment inulin is used as non-digestible oligosaccharide B. Inulin is a type of fructo-oligosaccharide wherein at least 75% of the glycosidic linkages are β(2,1) linkages. Typically, inulin has an average chain length between 8 and 60 monosaccharide units. A suitable fructo-oligosaccharide for use in the compositions of the present invention is commercially available under the trade name Raftiline®HP (Orafti). Other suitable sources are raftilose (Orafti), fibrulose and fibruline (Cosucra) and Frutafit and frutalose (Sensus).

Most preferred is a mixture of transgalacto-oligosaccharide with an average DP below 10, preferably below 6 and a fructo-oligosaccharide with an average DP above 7, preferably above 11, even more preferably above 20.

If the preterm formula comprises galacto- and fructo-oligosaccharides the weight ratio is preferably from 1/99 to 99/1, more preferably from 1/19 to 19/1, even more preferably from 1 to 19/1.

Preferably, the preterm formula in the present method or use comprises the non-digestible oligosaccharides selected from the group consisting of fructo-oligosaccharide and galacto-oligosaccharide, preferbly the preterm formula comprises at least galacto-oligosaccharide, prefebly the preterm formula comprises transgalacto-oligosaccharide and fructo-oligosaccharide.

The nutritional composition in the present method or use preferably comprises 0.05 to 20 wt.% total non-digestible oligosaccharide, more preferably 0.5 to 15 wt.%, even more preferably 1 to 10 wt.%, most preferably 2.0 to 10 wt.%, based on dry weight of the present composition.
Based on 100 ml the nutritional composition preferably comprises 0.01 to 2.5 wt.% total non-digestible oligosaccharide, more preferably 0.05 to 1.5 wt.%, even more preferably 0.25 to 1.5 wt.%, based on 100 ml of the present composition.

In one embodiment in the method or use according to the present invention the nutritional composition is a glutamine-based supplement, suitable to fortify human milk, to fortify human milk fortified with a standard human milk fortifier or to fortify a standard preterm formula. In the context of this invention, a supplement does not comprise all macro- and micronutrients needed for preterm infants so as to achieve a growth similar to fetal growth coupled with satisfactory functional development.

Standard preterm formula, and human milk fortified with standard human milk fortifier comprises all macro- and micronutrients needed for preterm infants so as to achieve a growth similar to fetal growth coupled with satisfactory functional development. In standard preterm formula, standard human milk fortifier, human milk and human milk fortified with standard human milk fortifier the amount of glutamine, more preferably free glutamine or glutamine in the form of a dipeptide, is below 12 wt.% based on total protein, also the amount of glutamine is below 1.5 wt.% based on dry weight, and also the amount of glutamine is below 0.3g per 100 kcal. The composition of a standard preterm formula is similar as described above, except for the glutamine amounts, which are lower.

The glutamine based supplement in the method or use according to the present invention is preferably packed in unit dose form. Preferably, the glutamine based supplement is present in a container. The container may contain more than one unit dose, preferably the container contains one single unit dose. Preferably the container is a sachet. Also preferably the container is a capsule or an ampoule. In a preferred embodiment the glutamine-based supplement is present in a container, in unit dose form, and in dry form, preferably in the form of a powder, and is present in an amount of from 0.05 to 10 g, preferably 0.05 g to about 8 g powder, preferably 0.1 to 4 g even more preferable 0.2 to 1 g powder per container. Preferably the container is provided with instructions for adding at least a portion of said glutamine-based supplement to human milk, to standard preterm formula, or to human milk fortified with standard HMF. Preferably, the nutritional supplement is a reconstitutable powder. Powdered nutritional supplements have the advantage that they minimize the volume displacement of preterm formula or human milk.

In one embodiment according to the method or use of the invention the nutritional composition is a glutamine-based supplement in the form of a powder in a unit dose comprising at least 15 wt.% glutamine in the form of free glutamine, glutamine dipeptide and/or glutamine tripeptide on dry weight of the powder.

Preferably the glutamine-based supplement in the present method or use comprises at least 15 wt.%, more preferably at least 20 wt.%, even more preferably at least 30 wt.% glutamine based on total protein, even more preferably more than 50 wt.%, most preferably more than 75 wt.% glutamine based on total protein. Preferably the glutamine-based supplement comprises at least 1.5 wt.%, more preferably at least 2 wt.%, more preferably at least 4 wt.% glutamine, even more preferably at least 15 wt.%, even more preferably at least 40 wt.%, most preferably at least 75 wt.% glutamine based on dry weight of the supplement. Preferably the glutamine-based supplement comprises at least 0.3 g, more preferably at least 0.5 g, more preferably at least 1 g glutamine, even more preferably at least 5, more preferably at least 10, most preferably at least 20 g glutamine based on 100 kcal.

Preferably the glutamine-based supplement in the present method or use comprises at least 15 wt.%, more preferably at least 20 wt.%, even more preferably at least 30 wt.% glutamine in the form of free amino acid and/or dipeptide based on total protein, even more preferably more than 50 wt.%, most preferably more than 75 wt.% glutamine in the form of free amino acid and/or dipeptide based on total protein. Preferably the glutamine-based supplement of the present invention comprises at least 1.5 wt.%, more preferably at least 2 wt.%, more preferably at least 4 wt.%, even more preferably at least 15 wt.%, even more preferably at least 40 wt.%, most preferably at least 75 wt.% glutamine in the form of free amino acid and/or dipeptide based on dry weight of the supplement. Preferably the glutamine-based supplement of the present invention comprises at least 0.3 g, more preferably at least 0.5 g, more preferably at least 1 g glutamine, even more preferably at least 5, more preferably at least 10, most preferably at least 20 g glutamine in the form of free amino acids and/or dipeptide based on 100 kcal.

In one embodiment, the glutamine-based supplement comprises further components such as proteins other than free glutamine, carbohydrates, fats, minerals like calcium and-or vitamins. In one embodiment, the glutamine-based supplement comprises non digestible oligosaccharides as discussed above.

Preferably the glutamine-based supplement is added to human milk, standard preterm formula, or human milk fortified with standard human milk fortifier in such an amount that the final glutamine enriched nutritional composition comprises at least 12 wt.%, more preferably at least 15 wt.%, more preferably at least 20 wt.%, even more preferably at least 30 wt.% glutamine based on total protein. Preferably the nutritional supplement is added to human milk, standard preterm formula, or human milk fortified with standard human milk fortifier in such an amount that the final glutamine enriched nutritional composition comprises at least 1.5 wt.%, more preferably at least 2 wt.%, even more preferably at least 4 wt.% glutamine based on dry weight of the nutritional composition. Preferably the nutritional supplement is added to human milk, standard preterm formula, or human milk fortified with standard human milk fortifier in such an amount that the final glutamine enriched nutritional composition comprises at least 0.3 g, more preferably at least 0.5 g, even more preferably at least 1 g glutamine based on 100 kcal.

Preferably the nutritional supplement is added to human milk, standard preterm formula, or human milk fortified with standard human milk fortifier in such an amount that the final glutamine enriched nutritional composition comprises at least 12 wt.%, more preferably at least 15 wt.%, more preferably at least 20 wt.%, even more preferably at least 30 wt.% glutamine in the form of free amino acid and/or dipeptide based on total protein. Preferably the nutritional supplement is added to human milk, standard preterm formula, or human milk fortified with standard human milk fortifier in such an amount that the final glutamine enriched nutritional composition comprises at least 1.5 wt.%, more preferably at least 2 wt.%, even more preferably at least 4 wt.% glutamine in the form of amino acid and/or dipeptide based on dry weight of the nutritional composition. Preferably the nutritional supplement is added to human milk, standard preterm formula, or human milk fortified with standard human milk fortifier in such an amount that the final glutamine enriched nutritional composition comprises at least 0.3 g, more preferably at least 0.5 g, even more preferably at least 1 g glutamine in the form of free amino acid and/or dipeptide based on 100 kcal. Preferably the glutamine-based supplement comprises at least 10 wt.% non digestible oligosaccharides based on dry weight of the glutamine-based supplement, preferably at least 15 wt.%, more preferably at least 20 wt.%, more preferably at least 25 wt.%, or at least 30 wt.% or at least 40 wt.% up to at most 50 wt.% non digestible oligosaccharides based on dry weight of the glutamine-based supplement.

Preferably, the non digestible oligosaccharides in the glutamine-based supplement invention are selected from the group consisting of fructo-oligosaccharide and galacto-oligosaccharide.

Preferably, the glutamine-based supplement comprises the non-digestible oligosaccharides transgalacto-oligosaccharide and fructo-oligosaccharide.

Preferably the nutritional composition comprising glutamine is in dry form, preferably a powder. This powder is suitable for reconstitution with water or another aqueous phase. When glutamine is in powder form it advantageously has a better shelf life. Glutamine, in particular free glutamine and glutamine dipeptide, is more stable when stored in dry form.

### Application

Administration of the nutritional composition to the very preterm infant born small for gestational age is enterally or parenterally, more preferably enterally, more preferably orally via a bottle, even more preferably enterally by naso-gastric tube feeding. Enteral feeding has the advantage that the glutamine will provide a beneficial effect in the intestinal tract as well regarding intestinal infections and improving barrier function.

Administration of the nutritional composition comprising glutamine is preferably during the first month after birth, more preferably until a body weight of 2500 is reached. Preferably the administration is daily. Preferably the administration is daily for at least a period of a week, more preferably 30 days.

The inventors found that glutamine enriched feeding in the first month after birth in preterm infant born small for gestational age has a long term effect on functional brain development. Advantageously an improvement in cognitive functioning was observed in children who were born preterm and were small for getational age and were supplemented in the first month after birth with glutamine when compared to the control group of preterm infants small for gestational age not having received glutamine. In particular a statistically significant increased in IQ was observed.

IQ stands for intelligence quotient and is a score derived from one of several standardized tests designed to assess intelligence.

The present invention preferably relates to improving cognitive functioning later in life, preferably at an age above 4 years, more preferably at an age between 4 to 12 years, and/or improving IQ later in life, preferably at an age above 4 years, more preferably at an age between 4 to 12 years, in preterm infants small for gestational age by adminstering a nutritional composition comprising glutamine to the infants in the first months of life. With improvement in cognitive functioning and improvement in IQ, an improvement compared to a preterm born small for gestational age control group not having received the nutritional composition comprising glutamine is meant.

The effects on cognitive functioning and/or IQ are preferably established later in life. The effects on cognitive functioning and/or IQ are preferably established during school age. With school age an age between 4 to 12 years is meant. It is particularly beneficial that the observed effects of the nutritional composition comprising glutamine on cognitive functioning are established during school age, since then the cognitive function is more evident.

### EXAMPLES

### Example 1: Effect of glutamine supplementation in VLBW and/or very preterm infants on brain volume later in life and on functional outcome in small for gestational age preterm children later in life

A randomized, placebo controlled clinical trial was performed. For a detailed protocol of the study, see van den Berg et al, 2004, Glutamine-enriched enteral nutrition in very low birth weight infants. Design of a double-blind randomised controlled trial [ISRCTN73254583], BMC Pediatrics 4:17.

In short: Infants with a gestational age <32 weeks and/or birth weight <1500 g were enrolled. In total 52 very preterm and/or VLBW children, which had taken part in a randomized controlled trial on enteral glutamine supplementation between day 3 and 30 of life, participated in this follow up study at the mean age of 8.6 years (SD = 0.3 years). 30 infants were in the control group and 22 in the glutamine supplementation group. Also comparisons were made with a control group (n=52) of term born infants.

The glutamine nutritional supplement was in powder form and contained 82 wt.% L-glutamine in total and 18 wt.% glucose (nitrogen 15.5 wt/wt%; 371 kcal/100 g), whereas the isonitrogenous control powder contained 100 wt.% L-alanine (nitrogen 15.7 wt/wt%, 435 kcal/100 g). The two powders were indistinguishable by appearance, colour and smell. Between days 3 and 30 of life, supplementation was administered in increasing doses to a maximum of 0.3 g/kg glutamine per day in the glutamine group. Initially, the supplementation dose was based on birth weight. After 2 weeks the dose was adjusted to actual body weight. Two members of the nursing staff daily added supplementation to breast milk or to preterm formula (Nenatal®, Nutricia Nederland B.V., Zoetermeer, The Netherlands), according to the parents' choice. Per 100 ml, Nenatal® provides 78 kcal, 2.1 g protein (casein-whey protein weight ratio 40:60), 4.4 g fat and 7.5 g carbohydrate. Nenatal® does not contain free glutamine and alanine or glutamine and alanine in the form of dipeptide. Protocol guidelines for the introduction of parenteral and enteral nutrition are disclosed in van den Berg et al., 2004.

All statistical analyses are performed on an intention to treat basis. In addition, alternative per protocol analyses are performed, excluding all patients who are not treated according to protocol, defined as more than 3 consecutive days or more than a total of 5 days on minimal enteral feeding or without supplementation. A p value <0.05 is considered significant (two-tailed). SPSS 9.0 (SPSS Inc., Chicago, IL, USA) and STAT 7.0 (Stat-Corp LP, College Station, TX, USA) are used for data analysis. A p value of 0.1 or below is considered to represent a realistic effect, a trend. Due to the small size of the groups also the effect size is taken into account. An effect size above 0.4 is considered to represent a trend.

### Results:

No statistically significant difference of the control preterm group and glutamine supplemented preterm group for follow up was present regarding base line characteristics, such as age, social economic status, ethnicity, birth weight, gestational age, head circumference, HELLP syndrome, BPD (bronchopulmonary dysplasia), IVH (intraventricular hemorrhage) grade I/II, IVH grade III/IV, ROP (retinopathy of prematurity) grade III/IV, Apgar score after 5 min., mode of delivery, gender and drop outs. The control group had more infections and a higher use of prenatal corticosteroids. Mean birth weight was 1186 (sd 336) gram in the control group, and 1252 (sd 380) in the glutamine group. Gestational age was 28.9 (sd 1.7) weeks in the control group and 29.4 (sd 1.7) in the glutamine group.

Brain cortical volumes were determined by MRI and calculated using FSL software "FAST" and corrected for age at MRI scan, gender and body weight for gestational age.
Brain cortical volumes were significantly higher in the term control group than in the preterm group. This was the case for intracranial volume, grey matter, white matter and cerebellum volume. In the preterm group, the glutamine supplemented group had a significantly higher white matter volume. A tendency for an increase in intracranial volume, and cerebellum volume was also observed, see Table 1. The cortical brain volumes in the glutamine group were higher than in the control group, and were more comparable to the term control group.

**Table 1: Cortical volumes in children born preterm and being supplemented with L-glutamine or with control during the first month of life.**

| Brain structures (in cm³) | Control | Glutamine | Term infants | Effect size* | P-value* |
|---|---|---|---|---|---|
| Intracranial volume (sd) | 1574.1 (134.4) | 1649.8 (179.3) | 1654.1 (132.7) | 0.48 | 0.07 |
| Grey matter (sd) | 705.4 (63.3) | 727.4 (85.60) | 735.8 (53.6) | 0.29 | 0.39 |
| White matter (sd) | 466.5 (49.2) | 496.5 (60.4) | 494.5 (49.6) | 0.54 | 0.03 |
| Cerebellum (sd) | 105.7(9.5) | 110.89 (15.3) | 113.2 (11.1) | 0.41 | 0.13 |

| | | | | | |
|---|---|---|---|---|---|
| *For control versus glutamine group | | | | | |

Brain subcortical volumes were determined by MRI and calculated using FSL software "FAST" and corrected for age at MRI scan, gender and body weight for gestational age. Brain subcortical volumes were significantly higher in the term controls than in preterm group. This was the case for the brain stem, thalamus, putamen, caudate nucleus, hippocampus, globus pallidum, amygdale, nucleus accumbens and the corpus callosum volume. In the preterm group, the glutamine supplemented group had higher subcortical volume, and significantly higher brain stem volume and hippocampus volume, and a tendency for an increase in other subcortical brain structures volume, see Table 2. The subcortical brain volumes in the glutamine group were higher than in the control group, and were more comparable to the term control group.

**Table 2: Subcortical volumes in children born preterm and being supplemented with L-glutamine or with control during the first month of life.**

| Brain structures (in cm³) | Control | Glutamine | Term infants | Effect size* | P-value* |
|---|---|---|---|---|---|
| Subcortical structures | 62.9(5.81) | 66.5(8.2) | 68.5(4.9) | 0.50 | 0.06 |
| - Brain stem | 17.6(2.2) | 18.9(2.5) | 19.2(1.8) | 0.54 | 0.04 |
| - Thalamus | 14.6(1.5) | 15.3(2.1) | 16.0(1.1) | 0.39 | 0.14 |
| - Putamen | 10.1(1.1) | 10.9(1.6) | 10.9(1.0) | 0.55 | 0.06 |
| - Caudate Nucleus | 7.3(1.1) | 7.4(1.4) | 8.0(1.0) | 0.10 | 0.86 |
| - Hippocampus | 6.9(0.6) | 7.2(0.8) | 7.4(0.8) | 0.47 | 0.04 |
| - Globus Pallidum | 3.3(0.3) | 3.5(0.4) | 3.5(0.3) | 0.48 | 0.10 |
| - Amygdala | 2.2(0.39) | 2.3(0.4) | 2.3(0.4) | 0.46 | 0.08 |
| - Nucleus accumbens | 1.0(0.18) | 1.0(0.3) | 1.1(0.2) | 0.04 | 0.82 |
| Corpus callosum (in mm²) | 521.0 (72.0) | 535.7 (90.0) | 558.4 (80.7) | 0.18 | 0.40 |

| | | | | | |
|---|---|---|---|---|---|
| *For control versus glutamine group | | | | | |

A subgroup of infants born small for gestational age (SGA <10^{th} percentile) was identified and analysed separately. Intellectual development was analysed in this subgroup of infants by a short form of the Wechsler Intelligence Scale for Children-III (WISC-III), including the subtests Vocabulary and Block Design. Both subtests correlate strongly (r 0·90) with full-scale intelligence quotient (FSIQ). Scores on this test were normalised with a mean of 100 (SD 15).

### Results

Preterm-SGA children had smaller brain volumes than appropriate for gestational age (AGA) children (*d*=1.36, p<.001).
A group of nine SGA infants that received placebo was compared with a group of nine SGA infants that received glutamine treatment.

There was a significant interaction between SGA status and glutamine group for total IQ score (p < 0.05). A significant improvement in IQ (WISC-III FSIQ being 102.3 sd 11.2, n=9) in the glutamine group compared to the control group (WISC-III FSIQ 81.7 sd 15.4 n=9) was found.
Preterm children born small for gestational age (SGA <10^{th} percentile) have relatively larger reductions in brain volumes. The increased brain volumes at school age after glutamine supplementation at birth are considered to be beneficial for improving cognitive function, in particular improving IQ, of SGA children with relatively small brain volumes at birth.

### Example 2: Preterm formula enriched in glutamine

Preterm formula in powder form comprising per 100 g about 486 kcal, 15.1 g protein, 46.1 g digestible carbohydrates, 26.7 g fat and 4.8 g non-digestible oligosaccharides. Furthermore the composition comprises minerals, trace elements, vitamins, L-carnitine, choline, myoinositol and taurine as known in the art and according to guidelines for preterm infants. For a ready to drink form, about 16.5 g powder is reconstituted with water until a final volume of 100 ml.
The protein comprises about 85 wt.% casein and whey protein from cow's milk based on total protein in a weight ratio of 1:1.5. About 15 wt.% of the protein is free glutamine.
The non-digestible oligosaccharides are galacto-oligosaccharides (Vivinal GOS) and fructopolysaccharides (RaftilinHP) in a weight ratio of 9:1.

### Example 3: Nutritional supplement enriched in glutamine.

Human milk fortifier in powder form packed in sachets comprising about 2 g.
Based on 100 g powder the composition comprises 361 kcal, 19 g protein, 71.5 g digestible carbohydrates, the rest being minerals, trace elements and vitamins. The protein consists of free glutamine.

## Claims

1. Glutamine, or a nutritional composition comprising glutamine, for use in improving cognitive functioning later in life in a very preterm infant born small for gestational age.

2. Glutamine, or a nutritional composition comprising glutamine, for use in improving IQ later in life in a very preterm infant born small for gestational age.

3. The glutamine or nutritional composition comprising glutamine for use according to claim 1 or 2 wherein the daily dose of glutamine provided to the infant is at least 0.01 to 0.5 g/kg body weight, preferably 0.03 to 0.4, even more preferably 0.07 to 0.35 g/kg body weight.

4. The glutamine or nutritional composition comprising glutamine for use according to any one of claims 1 to 3, wherein the nutritional composition is administered to the infant during the first month after birth.

5. The glutamine or nutritional composition comprising glutamine for use according to any one of claims 1 to 4, wherein the nutritional composition is a preterm formula.

6. The glutamine or nutritional composition comprising glutamine for use according to any one of claims 1 to 5, wherein the improvement in cognitive functioning is established at an age above 4 years, preferably at an age between 4 to 12 years.

7. The glutamine or nutritional composition comprising glutamine for use according to any one of claims 1 to 6, wherein the nutritional composition comprises between 1.5 wt.% and 20 wt.% glutamine based on dry weight of the nutritional composition, and comprises protein in such an amount that glutamine is present between 12 wt.% and 80 wt% based on total protein.

8. The glutamine or nutritional composition comprising glutamine for use according to any one of claims 1 to 7, wherein the glutamine in the nutritional composition is in the form of free glutamine, glutamine dipeptide and/or glutamine tripeptide, preferably free glutamine and/or glutamine dipeptide.

9. The glutamine or nutritional composition comprising glutamine for use according to any one of claims 1 to 8, wherein the nutritional composition further comprises non-digestible oligosaccharides selected from the group consisting of galacto-oligosaccharides and fructo-oligosaccharides.

10. The glutamine or nutritional composition comprising glutamine for use according to claim 9, wherein the nutritional composition comprises at least 0.5 wt.% non digestible oligosaccharides based on dry weight of the composition.

11. The glutamine or nutritional composition comprising glutamine for use according to any one of claims 9 or 10, wherein the nutritional composition comprises galacto-oligosaccharides.

12. The glutamine or nutritional composition comprising glutamine for use according to any one of claims 1 to 11 wherein the nutritional composition is a glutamine-based supplement in the form of a powder in a unit dose comprising at least 15 wt.% glutamine in the form of free glutamine, glutamine dipeptide and/or glutamine tripeptide based on dry weight of the powder or comprising at least 30 wt% glutamine based on total protein.

13. The glutamine or nutritional composition comprising glutamine for use according to claim 12 wherein the glutamine-based supplement comprises at least 10 wt.% non digestible oligosaccharides based on dry weight of the glutamine-based supplement.

## Patentansprüche

1. Glutamin, oder eine Nährstoffzusammensetzung, die Glutamin umfasst, zur Verwendung zum Verbessern des kognitiven Funktionierens im späteren Leben bei einem sehr frühen Frühgeborenen, das für das Gestationsalter zu klein geboren wurde.

2. Glutamin, oder eine Nährstoffzusammensetzung, die Glutamin umfasst, zur Verwendung zum Verbessern des IQ im späteren Leben bei einem sehr frühen Frühgeborenen, das für das Gestationsalter zu klein geboren wurde.

3. Glutamin oder Nährstoffzusammensetzung, die Glutamin umfasst, zur Verwendung gemäß Anspruch 1 oder 2, wobei die Tagesdosis von Glutamin, die dem Säugling bereitgestellt wird, wenigstens 0,01 bis 0,5 g/kg Körpergewicht, vorzugsweise 0,03 bis 0,4, noch stärker bevorzugt 0,07 bis 0,35 g/kg Körpergewicht beträgt.

4. Glutamin oder Nährstoffzusammensetzung, die Glutamin umfasst, zur Verwendung gemäß einem der Ansprüche 1 bis 3, wobei die Nährstoffzusammensetzung dem Frühgeborenen während des ersten Monats nach der Geburt verabreicht wird.

5. Glutamin oder Nährstoffzusammensetzung, die Glutamin umfasst, zur Verwendung gemäß einem der Ansprüche 1 bis 4, wobei die Nährstoffzusammensetzung eine Frühgeborenennahrung ist.

6. Glutamin oder Nährstoffzusammensetzung, die Glutamin umfasst, zur Verwendung gemäß einem der Ansprüche 1 bis 5, wobei die Verbesserung des kognitiven Funktionierens in einem Alter von mehr als 4 Jahren, vorzugsweise in einem Alter zwischen 4 bis 12 Jahren festgestellt wird.

7. Glutamin oder Nährstoffzusammensetzung, die Glutamin umfasst, zur Verwendung gemäß einem der Ansprüche 1 bis 6, wobei die Nährstoffzusammensetzung zwischen 1,5 Gew.-% und 20 Gew.-% Glutamin, bezogen auf das Trockengewicht der Nährstoffzusammensetzung, umfasst, und Protein in einer solchen Menge umfasst, dass Glutamin in einer Menge zwischen 12 Gew.-% und 80 Gew.-%, bezogen auf das Gesamtprotein, vorhanden ist.

8. Glutamin oder Nährstoffzusammensetzung, die Glutamin umfasst, zur Verwendung gemäß einem der Ansprüche 1 bis 7, wobei das Glutamin in der Nährstoffzusammensetzung in Form von freiem Glutamin, Glutamindipeptid und/oder Glutamintripeptid, vorzugsweise freiem Glutamin und/oder Glutamindipeptid vorliegt.

9. Glutamin oder Nährstoffzusammensetzung, die Glutamin umfasst, zur Verwendung gemäß einem der Ansprüche 1 bis 8, wobei die Nährstoffzusammensetzung außerdem unverdauliche Oligosaccharide, ausgewählt aus der Gruppe bestehend aus Galactooligosacchariden und Fructooligosacchariden, umfasst.

10. Glutamin oder Nährstoffzusammensetzung, die Glutamin umfasst, zur Verwendung gemäß Anspruch 9, wobei die Nährstoffzusammensetzung wenigstens 0,5 Gew.-% unverdauliche Oligosaccharide, bezogen auf das Trockengewicht der Zusammensetzung, umfasst.

11. Glutamin oder Nährstoffzusammensetzung, die Glutamin umfasst, zur Verwendung gemäß einem der Ansprüche 9 oder 10, wobei die Nährstoffzusammensetzung Galactooligosaccharide umfasst.

12. Glutamin oder Nährstoffzusammensetzung, die Glutamin umfasst, zur Verwendung gemäß einem der Ansprüche 1 bis 11, wobei die Nährstoffzusammensetzung ein Ergänzungsmittel auf Glutaminbasis in Form eines Pulvers in einer Dosiseinheit ist, die wenigstens 15 Gew.-% Glutamin in Form von freiem Glutamin, Glutamindipeptid und/oder Glutamintripeptid, bezogen auf das Trockengewicht des Pulvers, umfasst oder wenigstens 30 Gew.-% Glutamin, bezogen auf das Gesamtprotein, umfasst.

13. Glutamin oder Nährstoffzusammensetzung, die Glutamin umfasst, zur Verwendung gemäß Anspruch 12, wobei das Ergänzungsmittel auf Glutaminbasis wenigstens 10 Gew.-% unverdauliche Oligosaccharide, bezogen auf das Trockengewicht des Ergänzungsmittels auf Glutaminbasis, umfasst.

## Revendications

1. Glutamine, ou composition nutritionnelle comprenant de la glutamine pour utilisation dans l'amélioration du fonctionnement cognitif plus tard dans la vie d'un nourrisson très prématuré né petit pour l'âge gestationnel.

2. Glutamine, ou composition nutritionnelle comprenant de la glutamine, pour une utilisation dans l'amélioration du QI plus tard dans la vie d'un nourrisson très prématuré né petit pour l'âge gestationnel.

3. Glutamine ou composition nutritionnelle comprenant de la glutamine pour utilisation selon la revendication 1 ou 2 dans laquelle la dose quotidienne de glutamine fournie au nourrisson est d'au moins 0.01 à 0.5 g/kg de poids corporel, de préférence de 0.03 à 0.4, encore plus préférablement de 0.07 à 0.35 g/kg de poids corporel.

4. Glutamine ou composition nutritionnelle comprenant de la glutamine pour utilisation selon l'une quelconque des revendications 1 à 3, dans laquelle la composition nutritionnelle est administrée au nourrisson durant le premier mois après la naissance.

5. Glutamine ou composition nutritionnelle comprenant de la glutamine pour utilisation selon l'une quelconque des revendications 1 à 4, dans laquelle la composition nutritionnelle est une formule pour prématuré.

6. Glutamine ou composition nutritionnelle comprenant de la glutamine pour utilisation selon l'une quelconque des revendications 1 à 5, dans laquelle l'amélioration de la fonction cognitive est établie à un âge supérieur à 4 ans, de préférence à un âge compris entre 4 et 12 ans.

7. Glutamine ou composition nutritionnelle comprenant de la glutamine pour utilisation selon l'une quelconque des revendications 1 à 6, dans laquelle la composition nutritionnelle comprend entre 1.5% en poids et 20% en poids de glutamine par rapport au poids sec de la composition nutritionnelle, et comprend une protéine en quantité telle que la glutamine est présente entre 12% en poids et 80% en poids, sur la base de la protéine totale.

8. Glutamine ou composition nutritionnelle comprenant de la glutamine pour utilisation selon l'une quelconque des revendications 1 à 7, dans laquelle la glutamine dans la composition nutritionnelle se présente sous la forme de glutamine libre, dipeptide de glutamine et/ou tripeptide de glutamine, de préférence glutamine libre et/ou dipeptide de glutamine.

9. Glutamine ou composition nutritionnelle comprenant de la glutamine pour utilisation selon l'une quelconque des revendications 1 à 8, dans laquelle la composition nutritionnelle comprend en outre des oligosaccharides non digestibles choisis dans le groupe constitué par les galacto-oligosaccharides et fructo-oligosaccharides.

10. Glutamine ou composition nutritionnelle comprenant de la glutamine pour utilisation selon la revendication 9, dans laquelle la composition nutritionnelle comprend au moins 0.5% d'oligosaccharides non digestibles sur la base du poids sec de la composition.

11. Glutamine ou composition nutritionnelle comprenant de la glutamine pour utilisation selon l'une quelconque des revendications 9 ou 10, dans laquelle la composition nutritionnelle comprend des galacto-oligosaccharides.

12. Glutamine ou composition nutritionnelle comprenant de la glutamine pour utilisation selon l'une quelconque des revendications 1 à 11, dans laquelle la composition nutritionnelle est un suppplément à base de glutamine sous forme d'une poudre dans une dose unitaire comprenant au moins 15% en poids de glutamine sous la forme de glutamine libre, dipeptide de glutamine et/ou tripeptide de glutamine par rapport au poids sec de la poudre ou comprenant au moins 30% en poids de glutamine sur la base de la protéine totale.

13. Glutamine ou composition nutritionnelle comprenant de la glutamine pour utilisation selon la revendication 13, dans laquelle le suppplément à base de glutamine comprend au moins 10% en poids d'oligosaccharides non digestibles par rapport au poids sec du suppplément à base de glutamine.
